# EUROPEAN PATENT SPECIFICATION

(11) **EP 2 723 407 B1**
(45) Date of publication and mention of the grant of the patent: **19.07.2017**
(21) Application number: 12731841.8
(22) Date of filing: 19.06.2012
(51) Int. Cl.: A61L 15/28, A61L 15/30, A61L 15/34, A61L 15/40, A61L 15/44, A61L 15/58, A61L 15/60, A61L 24/00, A61L 24/04, A61Q 19/00, A61K 8/02, A61K 8/73, A61K 8/81, A61K 8/92, A61K 8/97, A61K 9/70

(54) **ADHESIVE OR HYDROCOLLOID CONTAINING VEGETABLE OIL**
KLEBSTOFF ODER HYDROKOLLOID MIT PFLANZLICHEM ÖL
HUILE VÉGÉTALE CONTENANT UN ADHÉSIF OU UN HYDROCOLLOÏDE

(30) Priority: 24.06.2011 US 201161500709 P
(43) Date of publication of application: 30.04.2014
(73) Proprietor: Avery Dennison Corporation, Glendale, CA 91203 (US)
(72) Inventor: WIBAUX, Anne Marie, B-2018 Antwerpen (BE); VAN DE POL, Vicky, B-2300 Turnhout (BE)
(74) Representative: Müller-Boré & Partner Patentanwälte PartG mbB
(86) International application number: PCT/US2012/043070
(87) International publication number: WO 2012/177590

(56) References cited:
- WO-A1-01/13968
- WO-A1-01/30406
- WO-A1-2011/044449
- US-A1- 2004 071 757
- US-A1- 2004 241 215
- US-B1- 6 190 689

## Description

### CROSS REFERENCES TO RELATED APPLICATIONS

The present application claims the benefit of U.S. Provisional Patent Application No. 61/500,709 filed June 24, 2011.

### FIELD

The present subject matter relates to an adhesive or hydrocolloid composition that contains vegetable oil.

### BACKGROUND

Adhesive compositions are known which contain oils. Mineral oil is widely used as a plasticizer for compounding hot melt adhesives and hydrocolloids. However, for applications in which adhesives may contact skin, mineral oil may produce or aggravate acne. Thus, mineral oil is not acceptable for use in adhesives for many medical and consumer health care applications.

Although a wide array of plasticizers are known, only a limited number are acceptable for incorporation in adhesives used in medical and consumer health care applications. Of the plasticizers that are acceptable for these applications, many are costly and/or limited in availability.

Accordingly, a need remains for an adhesive suitable for medical and health care applications which overcomes the disadvantages of adhesives containing mineral oil. And, a need exists for a plasticizer that is relatively inexpensive, widely available, and which is acceptable for medical and health care applications.

### SUMMARY

The difficulties and drawbacks associated with previously known compositions and products are addressed in the present compositions, methods and articles relating to an adhesive or hydrocolloid composition comprising one or more vegetable oils.

The subject matter of the present invention is defined in claims 1-9 as attached. Embodiments described herein which are not covered by the claims merely serve to illustrate the technical context of the present invention.

In a first aspect, the present subject matter provides a composition comprising, by weight: (i) from 0.1 % to 20 % of at least one vegetable oil; (ii) from 37.5 % to 60 % of hot melt adhesive(s) containing styrene-isoprene-styrene and styrene-isoprene block copolymers; (iii) from 30 % to 35 % of one or more hydrocolloid(s); and (iv) from 0 to 17.5 % of polyisobutene.

In another aspect, the present subject matter provides a method of preparing a composition including vegetable oil according the first aspect, the method comprising: providing the components (i) to (iii) and optionally (iv) given in the first aspect in the amounts given in the first aspect, and blending component (i) with components (ii) and (iii) and optionally (iv) to thereby form the composition.

In yet another aspect, the subject matter provides an article adapted for placement on a user's skin. The article defines a skin-contacting region, wherein a composition according to the first aspect is disposed on at least a portion of the skin-contacting region.

As will be realized, the present subject matter is capable of other and different embodiments and its several details are capable of modifications in various respects, all without departing from the subject matter. Accordingly, the drawings and description are to be regarded as illustrative and not restrictive.

### BRIEF DESCRIPTION OF THE DRAWINGS

Figure 1 is a graph of 90° peel adhesion results of samples using the preferred embodiment compositions.
Figure 2 is a graph of reverse tack results of samples using the preferred embodiment compositions.
Figure 3 is a graph of static absorption of samples using the preferred embodiment compositions.

### DETAILED DESCRIPTION OF THE EMBODIMENTS

The present subject matter relates to adhesives and/or hydrocolloid compositions that comprise one or more vegetable oils. The resulting compositions provide a soothing effect when applied to skin and may further provide anti-infective properties. The vegetable oil containing compositions also assist healing of wounds or other tissue damage by promoting the formation of scar tissue. This characteristic is known as cicatrisation. The incorporation of vegetable oil has also been found to provide a plasticizing effect upon the resulting compositions.

In addition to various compositions, the present subject matter also provides methods for preparing the compositions and methods of using the compositions.

The present subject matter additionally provides various products and namely medical products and consumer health care products using the compositions. These and other aspects are described in greater detail herein.

### Vegetable Oil(s)

A wide array of vegetable oils can be utilized in accordance with the present subject matter. Generally, any vegetable oil or fat derived from plants can be used. Such oils or fats are lipid materials. Generally, the distinction between oils and fats is that oils are liquid at room temperature, while fats are solid at room temperature. Both vegetable oils and fats include triglycerides. The term "vegetable oil(s)" as used herein refers to both vegetable fats and vegetable oils.

Vegetable oils comprise triglycerides which are esters that include three fatty acids bound to a glycerol molecule. Examples of vegetable oils include, but are not limited to, soybean oil, palm oil, olive oil, corn oil, grape seed oil, borage oil, peanut oil, tung oil, canola oil, linseed oil, rapeseed oil, almond oil (also known as amandel oil), castor oil, coconut oil, cottonseed oil, palm kernel oil, rice bran oil, argan oil, safflower oil, sesame oil, sunflower oil, avocado oil, jojoba oil, tall oil, and combinations thereof. Typically, the fatty acids associated with vegetable oils include long chain, e.g. C₈ to C₂₂ and more typically C₁₂ to C₁₄, moieties, many of which include multiple double bonds per chain. The glycerol molecule has three hydroxyl (OH-) groups. Each fatty acid has a carboxyl group (COOH-). In triglycerides, the hydroxyl groups of the glycerol join the carboxyl groups of the fatty acids to form ester bonds.

As noted, chain lengths of the fatty acids in naturally occurring or bio-based triglycerides can be of varying lengths. However, chain lengths having 16, 18, and 20 carbons are the most common. Natural fatty acids found in plants are typically composed only of even numbers of carbon atoms as a result of how they are bio-synthesized from acetyl coenzyme A.

Most natural fats contain a complex mixture of individual triglycerides. Because of this, most natural fats melt over a broad range of temperatures. Cocoa butter is unusual in that it is composed of only a few triglycerides, one of which contains palmitic, oleic, and stearic acids, in order of concentration. As a result, cocoa butter has a relatively narrow melting temperature range.

Preferred fatty acids in the triglycerides of the vegetable oils of interest are set forth below in Table 1.

**Table 1 - Chemical Names and Descriptions of Common Fatty Acids**

| Common Name | Carbon Atoms | Double Bonds | Scientific Name | Sources |
|---|---|---|---|---|
| Butyric acid | 4 | 0 | butanoic acid | butterfat |
| Caproic Acid | 6 | 0 | hexanoic acid | butterfat |
| Caprylic Acid | 8 | 0 | octanoic acid | coconut oil |
| Capric Acid | 10 | 0 | decanoic acid | coconut oil |
| Lauric Acid | 12 | 0 | dodecanoic acid | coconut oil |
| Myristic Acid | 14 | 0 | tetradecanoic acid | palm kernel oil |
| Myristoleic | 14 | 1 | 9-tetradecenoic acid | |
| Palmitic Acid | 16 | 0 | hexadecanoic acid | palm oil |
| Palmitoleic Acid | 16 | 1 | 9-hexadecenoic acid | |
| Stearic Acid | 18 | 0 | octadecenoic acid | |
| Oleic Acid | 18 | 1 | 9-octadecenoic acid | olive oil |
| Ricinoleic acid | 18 | 1 | 12-hydroxy-9-octadecenoic acid | castor oil |
| Vaccenic Acid | 18 | 1 | 11-octadecenoic acid | butterfat |
| Linoleic Acid | 18 | 2 | 9,12-octadecadienoic acid | grape seed oil |
| Alpha-Linolenic Acid (ALA) | 18 | 3 | 9,12,15-octadecatrienoic acid | flaxseed (linseed) oil |
| Gamma-Linolenic Acid (GLA) | 18 | 3 | 6,9,12-octadecatrienoic acid | borage oil |
| Arachidic Acid | 20 | 0 | eicosanoic acid | peanut oil |
| Gadoleic Acid | 20 | 1 | 9-eicosenoic acid | |
| Arachidonic Acid (AA) | 20 | 4 | 5,8,11,14-eicosatetraenoic acid | |
| EPA | 20 | 5 | 5,8,11,14,17-eicosapentaenoic acid | |
| Behenic acid | 22 | 0 | docosanoic acid | rapeseed oil |
| Erucic acid | 22 | 1 | 13-docosenoic acid | rapeseed oil |
| DHA | 22 | 6 | 4,7,10,13,16,19-docosahexaenoicacid | |
| Lignoceric acid | 24 | 0 | tetracosanoic acid | small amounts in most fats |

Vegetable oils contain varying amounts of triglycerides depending upon the type or source of the oil, and the ratio of oil to fat. See Tables 1 and 2, and "The Chemistry of Oils and Fats" by Frank D. Gunstone (Blackwell Publishing 2004). Table 2 set forth below, lists typical fatty acid amounts (as percentages by weight) of various common oils and fats.

**Table 2 - Typical Fatty Acid Composition (wt%) of Common Oils and Fats**

| Oil/Fat | 16:0 | 16:1 | 18:0 | 18:1 | 18:2 | 18:3 | 20:1 | 22:1 | 24:0 | Average Unsaturation Per Triglyceride |
|---|---|---|---|---|---|---|---|---|---|---|
| soybean | 11 | 0.1 | 4 | 23.4 | 53.2 | 7.8 | | | | 4.6 |
| palm | 44.4 | 0.2 | 4.1 | 39.3 | 10 | 0.4 | | | | 1.8 |
| rapeseed | 3 | 0.2 | 1 | 13.2 | 13.2 | 9 | 9 | 49.2 | 1.2 | 3.8 |
| sunflower | 6 | | 5 | 20 | 60 | | | | | 1.4 |
| tallow | 27 | 11 | 7 | 48 | 2 | | | | | 0.6 |
| cottonseed | 21.6 | 0.6 | 2.6 | 18.6 | 54.5 | 0.7 | | | | 3.9 |
| olive | 13.7 | 1.2 | 2.5 | 71.1 | 10 | 0.6 | | | | 2.8 |
| corn | 10.9 | 0.2 | 2 | 25.4 | 59.6 | 1.2 | | | | 4.5 |
| canola | 4.1 | 0.3 | 1.8 | 60.9 | 21 | 8.8 | 1 | 0.7 | 0.2 | 3.9 |
| linseed | 5.5 | | 3.5 | 19.1 | 15.3 | 56.6 | | | | 6.6 |

The unsaturation associated with the various triglycerides in the oils and/or fats serves as a potential reaction site for polymerization and/or for reaction. The double bonds are relatively unreactive in regards to polymerization unless conjugated as in drying oils such as Tung oil.

### Adhesive and Hydrocolloid

A wide range of adhesives and hydrocolloids may be used in the preferred embodiment compositions. According to the invention, the adhesive is a hot melt adhesive. Nonlimiting examples of suitable adhesives for use in the present subject matter include those described in EP 1206290, EP 1221986 (or its equivalent US 6,740,711), and EP 1383548 (or its equivalent US 7,335,416).

A preferred adhesive is a hot melt adhesive based on a styrene-containing thermoplastic elastomer and a liquid rubber, a low molecular weight polyisobutylene and a low molecular weight liquid polybutene, which forms a continuous phase. Dispersed within the continuous phase the adhesive also includes a discontinuous phase of one or more water soluble and/or water swellable absorbent polymers.

Suitable styrene-containing thermoplastic elastomers include block copolymers based on styrene-butadiene, styrene-isoprene or styrene ethylene-butylene. Also, a low styrene synthetic copolymer of butadiene and styrene, commonly called SBR rubber, can be used as the thermoplastic elastomer. The elastomer may consist of linear or radial A-B-A block copolymers or mixtures of these A-B-A copolymers with simple A-B block copolymers. However, the proportion of A-B block copolymers in the mixture of A-B-A and A-B block copolymers should not exceed about 85% by weight and lower percentages would normally be used.

The A-B-A block copolymers are of the type which consist of A blocks derived from styrene or one of its homologues and B blocks derived from conjugated dienes, such as butadiene or isoprene, or from lower alkenes such as ethylene or butylene. The radial A-B-A polymers useful in this subject matter are of the type described for example in U.S. Pat. No. 3,281,383 and conform to the general formula (A-B) ₙ X, where A and B comprise blocks derived from monomers described above in connection with the A-B-A copolymers, X is an organic or inorganic connecting moiety having a functionality of at least 2, and n is equal to the functionality of X. The A-B block copolymers useful in this subject matter comprise A and B blocks derived from monomers described above in connection with the A-B-A copolymers.

Liquid rubbers include synthetic liquid isoprene rubber, depolymerised natural rubber, carboxyl terminated synthetic liquid isoprene-styrene rubber, hydroxyl terminated synthetic liquid isoprene rubber, hydrogentated liquid isoprene rubber, liquid isoprene-styrene copolymer, liquid isoprene-butadience copolymer and liquid butadiene-styrene copolymer. The liquid rubbers have a molecular weight of about 25,000 to about 50,000. Preferably, the liquid rubbers have a glass transition temperature of less than -50°C, and a melt viscosity at 38°C of from 500-10,000 poises. A particularly preferred material for use as the liquid rubber component is a blend of 80% styrene isoprene liquid rubber and 20% styrene isoprene rubber. All references to percentages are percentages by weight. It will be appreciated that other liquid rubbers known in the art could be useful. According to the invention, the hot melt adhesive contains styrene-isoprene-styrene and styrene-isoprene block copolymers.

The polyisobutylene component is exemplified by the VISTANEX LM series of polyisobutylenes, available from Exxon Chemical Corporation, and which have Flory viscosity average molecular weights in the range 35,000 to 70,000, and Brookfield viscosities at 175° C within the range 20,000 and 140,000 mPa·s. Another preferred polyisobutene is commercially available from BASF under the designation OPPANOL B, and preferably B12. However, OPPANOL is available in various molecular weights and depending upon the particular application, any grade such as from grade B11 to B15, could be used.

The low molecular weight polybutene components are exemplified by the HYVIS series of materials from BP, and by the PARAPOL series of products from Exxon Chemical Corporation, and which have molecular weights ranging from 1000 to 3000, determined using test method AM-I 841-86, and kinematic viscosities at 100° C within the range 180 and 3500 cSt, as measured by test method ASTM D445.

Typically, a suitable processing stabilizer is also included in the hot melt adhesive component. Suitable stabilizers useful include those indicated for use with styrene-olefin styrene block copolymer thermoplastic elastomers such as organophosphites and the so-called hindered phenols, but any suitable stabilizers may be employed. An example of an organophosphite stabilizer is tris(nonylphenyl) phosphite, available as POLYGARD HR manufactured by Uniroyal. Particularly useful are the hindered phenols, IRGANOX 1010 and IRGANOX 565, manufactured by Ciba. IRGANOX 1010 is pentaerythritol tetrakis (3-(3, 5-di-tert-butyl-4-hydroxyphenyl) propionate. IRGANOX 565 is 2, 6-di-tert-butyl-4-(4,6-bis(octylthio)-I, 3,5-triazin-2-ylamino)phenol. Stabilizers may be used or in combination, and suitable ranges are from 0.3 1.5% by weight based on the total formulation.

Other optional ingredients such as tackifiers and plasticizers may be added to the continuous phase, to modify tack and optimise adhesion properties.

The hot melt adhesive component is comprised of the thermoplastic elastomer and the liquid rubber, both as defined above. Preferably this hot melt adhesive is formed with a weight ratio of thermoplastic elastomer to liquid rubber of about 1:0.5 to about 1:7. The amount of liquid rubber used is varied for the desired degree of adhesiveness and tackiness of the pressure sensitive adhesive.

In certain applications, the present subject matter is based upon a hydrocolloid that comprises one or more vegetable oils. As will be appreciated, a hydrocolloid is a colloid system in which the colloid particles are dispersed in water. An example of a suitable hydrocolloid is carboxymethyl cellulose. Additional examples of suitable hydrocolloids include gelatin, pectin, alginate, or synthetic super absorbent hydrocolloids. Preferred hydrocolloids are hydrocolloids typically used in wound care applications. Examples of commercially available products containing hydrocolloid compositions include, but are not limited to, TEGASORB and TEGASORB THIN from 3M; HYDROCOL from Bertek (Dow Hickam); BGC Matrix from Brennan; COMFEEL available from Colorplast; DUODERM and SIGNADRESS from Convatec; DERMAFILM from DermaRite; RESTORE from Hollister; NU-DERM available from Johnson and Johnson; ULTEC available from Kendall; EXUDERM available from Medline; and REPLICARE, CUTINOVA HYDRO, and CUTINOVA THIN available from Smith & Nephew. Another example of a suitable hydrocolloid is sodium carboxymethyl cellulose available commercially under the designation AQUASORB from a variety of suppliers. It will be appreciated that in no way is the present subject matter limited to any of these hydrocolloids.

### Preferred Compositions

According to the invention, the compositions generally comprise from about 0.1% to about 20% of vegetable oil(s). Generally, if more than 20% vegetable oil is used in the composition, the composition is very uncohesive and if applied to human skin, tends to leave residue on the skin. All percentages noted herein are percentages by weight unless indicated otherwise.

The preferred embodiment compositions may also comprise one or more additional agents or components such as absorbent fillers, clays, antimicrobial agents, agents for treating pain, other medicinal agents and the like. The preferred embodiment compositions, particularly when comprising an adhesive component, exhibit sufficient tack and adhering ability to thereby enable their use in a wide range of medical and consumer health care applications.

A preferred component for including in the various adhesive and/or hydrocolloid compositions described herein is carboxymethyl cellulose (CMC). A variety of different grades of this material is commercially available. A preferred material is AQUASORB A500 available from Ashland Chemical. The A500 material is crystalline sodium carboxymethyl cellulose. This material or a suitable equivalent is also available from Aqualon, a division of Hercules Chemical.

One or more solvents can also be added. A wide array of solvents can be used such as water or oil based solvents. Preferred oil based solvents include, but are not limited to heptane or toluene. In addition, one or more surfactants can also be included.

According to the invention, the compositions comprise (i) from 0.1% to 20% and more preferably from 10% to 20% of one or more vegetable oil(s), (ii) from 37.5% to 60% of hot melt adhesive(s) containing styrene-isoprene-styrene and styrene-isoprene block copolymers, (iii) from 30% to 35% of one or more hydrocolloids such as sodium carboxymethyl cellulose, commercially available under the designation AQUASORB, and (iv) from 0 to 17.5% polyisobutene.

### Methods

The preferred compositions are preferably prepared by incrementally combining the one or more vegetable oil(s) with one or both of the adhesive component and the hydrocolloid component. Most preferably, the vegetable oil is added when in a liquid or flowable form. Heating may be used as necessary to transform the vegetable oil into a liquid or flowable form. The adhesive and/or hydrocolloid component is also preferably in a liquid or flowable form however, semi-viscous forms can also be used. It is contemplated that generally when using an adhesive component, the adhesive is heated to a temperature of from about 60°C to about 100° C. Preferably, the processing temperature is less than 70° C. The heated adhesive and/or hydrocolloid components are then blended with the one or more vegetable oil(s) in conventional mixing equipment. For example, a Z-Blade mixer can be used.

### Products and Devices

The preferred embodiment compositions can be used in a wide array of products and devices. Non-limiting examples of such products and devices include consumer patches, consumer wound care products, dressings, bandages, and cosmetic patches. The term "cosmetic patch" as used herein refers to a patch that is typically used to cover skin defects or improve skin condition(s). For example, anti-wrinkle eye patches available from Montagne & Jeunesre are an example of cosmetic patches. Another use for a cosmetic patch is a patch that is placed over acne. An example of such patch is a patch available from Neutrogena under the designation ON THE SPOT. The term "consumer patch" as used herein refers to a dressing typically used for small wound treatment such as products commercially available under the designation NAND-AID. The subject matter includes other types of articles which may employ the preferred embodiment compositions.

### Evaluations

A series of investigations were undertaken to further evaluate the preferred embodiment compositions.

A collection of preferred embodiment compositions were prepared as follows. The components of each composition or formulation were mixed or blended with one another. Blending was performed over the course of several phases with a total mixing time of about 36 minutes. The blending temperature was 60° C. Generally, during phase 1, blending was performed for 5 minutes in an open mixer. Phase 2 consisted of blending for 5 minutes in the open mixer. Phase 3 consisted of 5 minutes in the mixer however, closed. Tables 3-14 summarize the compositions. The components T2561 and T2560 are both hot melt adhesives containing styrene-isoprene-styrene and styrene-isoprene block copolymers. Specifically, T2560 contains 80% styrene-isoprene and 20% styrene-isoprene-styrene. The triblock copolymer of styrene-isoprene-styrene is KRATON D-1161 avilable from Kraton Polymers of Houston, Texas. T2561 contains 50% styrene-isoprene and 50% of styrene-isoprene-styrene. The triblock copolymer of styrene-isoprene-styrene used in T2561 is KRATON 1117.

**Table 3 - Formulation A**

| RAW MATERIAL | TOTAL | | PHASE 1 | PHASE 2 | PHASE 3 | PHASE 4 |
|---|---|---|---|---|---|---|
| | | | | | | |
| Oppanol B12 | 17.5% | | 87.5 | | | |
| T2561 | 0.0% | | 0.00 | | | |
| T2560 | 37.5% | | | 187.50 | | |
| Aquasorb A500 | 35.0% | | | | 175.00 | |
| olive oil | 10.0% | | | | | 50.00 |
| | | | | | | |
| | | | | | | |
| | | | | | | |
| TOTAL | 100% | | 87.50 | 187.50 | 175.00 | 50.00 |
| MIXING TIME (min) | 36 | | 3 | 3 | 30 | |

**Table 4 - Formulation B**

| RAW MATERIAL | TOTAL | | PHASE 1 | PHASE 2 | PHASE 3 | PHASE 4 |
|---|---|---|---|---|---|---|
| | | | | | | |
| Oppanol B12 | 12.5% | | 62.5 | | | |
| T2561 | 0.0% | | 0.00 | | | |
| T2560 | 37.5% | | | 187.50 | | |
| Aquasorb A500 | 30.0% | | | | 150.00 | |
| olive oil | 20.0% | | | | | 100.00 |
| | | | | | | |
| | | | | | | |
| | | | | | | |
| TOTAL | 100% | | 62.50 | 187.50 | 150.00 | 100.00 |
| MIXING TIME (min) | 36 | | 3 | 3 | 30 | |

**Table 5 - Formulation C**

| RAW MATERIAL | TOTAL | | PHASE 1 | PHASE 2 | PHASE 3 | PHASE 4 |
|---|---|---|---|---|---|---|
| | | | | | | |
| Oppanol B12 | 0.0% | | 0 | | | |
| T2561 | 0.0% | | 0.00 | | | |
| T2560 | 60.0% | | | 300.00 | | |
| Aquasorb A500 | 30.0% | | | | 150.00 | |
| olive oil | 10.0% | | | | | 50.00 |
| | | | | | | |
| | | | | | | |
| | | | | | | |
| TOTAL | 100% | | 0.00 | 300.00 | 150.00 | 50.00 |
| MIXING TIME (min) | 36 | | 3 | 3 | 30 | |

**Table 6 - Formulation D**

| RAW MATERIAL | TOTAL | | PHASE 1 | PHASE 2 | PHASE 3 | PHASE 4 |
|---|---|---|---|---|---|---|
| | | | | | | |
| Oppanol B12 | 0.0% | | 0 | | | |
| T2561 | 30.0% | | 150.00 | | | |
| T2560 | 30.0% | | | 150.00 | | |
| Aquasorb A500 | 30.0% | | | | 150.00 | |
| olive oil | 10.0% | | | | | 50.00 |
| | | | | | | |
| | | | | | | |
| | | | | | | |
| TOTAL | 100% | | 150.00 | 150.00 | 150.00 | 50.00 |
| MIXING TIME (min) | 36 | | 3 | 3 | 30 | |

**Table 7 - Formulation E**

| RAW MATERIAL | TOTAL | | PHASE 1 | PHASE 2 | PHASE 3 | PHASE 4 |
|---|---|---|---|---|---|---|
| | | | | | | |
| Oppanol B12 | 0.0% | | 0 | | | |
| T2561 | 0.0% | | 0.00 | | | |
| T2560 | 60.0% | | | 300.00 | | |
| Aquasorb A500 | 30.0% | | | | 150.00 | |
| amandel oil | 10.0% | | | | | 50.00 |
| | | | | | | |
| | | | | | | |
| | | | | | | |
| TOTAL | 100% | | 0.00 | 300.00 | 150.00 | 50.00 |
| MIXING TIME (min) | 36 | | 3 | 3 | 30 | |

**Table 8 - Formulation F**

| RAW MATERIAL | TOTAL | | PHASE 1 | PHASE 2 | PHASE 3 | PHASE 4 |
|---|---|---|---|---|---|---|
| | | | | | | |
| Oppanol B12 | 0.0% | | 0 | | | |
| T2561 | 60.0% | | 300.0 | | | |
| T2560 | 0.0% | | | 0 | | |
| Aquasorb A500 | 30.0% | | | | 150.00 | |
| amandel oil | 10.0% | | | | | 50.00 |
| | | | | | | |
| | | | | | | |
| | | | | | | |
| TOTAL | 100% | | 0.00 | 300.00 | 150.00 | 50.00 |
| MIXING TIME (min) | 36 | | 3 | 3 | 30 | |

**Table 9 - Formulation G**

| RAW MATERIAL | TOTAL | | PHASE 1 | PHASE 2 | PHASE 3 | PHASE 4 |
|---|---|---|---|---|---|---|
| | | | | | | |
| Oppanol B12 | 0.0% | | 0 | | | |
| T2561 | 40.0% | | 200.00 | | | |
| T2560 | 20.0% | | | 100.00 | | |
| Aquasorb A500 | 30.0% | | | | 150.00 | |
| amandel oil | 10.0% | | | | | 50.00 |
| | | | | | | |
| | | | | | | |
| | | | | | | |
| TOTAL | 100% | | 200.00 | 100.00 | 150.00 | 50.00 |
| MIXING TIME (min) | 36 | | 3 | 3 | 30 | |

**Table 10 - Formulation H**

| RAW MATERIAL | TOTAL | | PHASE 1 | PHASE 2 | PHASE 3 | PHASE 4 |
|---|---|---|---|---|---|---|
| | | | | | | |
| Oppanol B12 | 0.0% | | 0 | | | |
| T2561 | 34.0% | | 170.00 | | | |
| T2560 | 20.0% | | | 100.00 | | |
| Aquasorb A500 | 34.0% | | | | 170.00 | |
| amandel oil | 12.0% | | | | | 60.00 |
| | | | | | | |
| | | | | | | |
| | | | | | | |
| TOTAL | 100% | | 170.00 | 100.00 | 170.00 | 60.00 |
| MIXING TIME (min) | 36 | | 3 | 3 | 30 | |

**Table 11 - Formulation I**

| RAW MATERIAL | TOTAL | | PHASE 1 | PHASE 2 | PHASE 3 | PHASE 4 |
|---|---|---|---|---|---|---|
| | | | | | | |
| Oppanol B12 | 0.0% | | 0 | | | |
| T2561 | 34.0% | | 170.00 | | | |
| T2560 | 20.0% | | | 100.00 | | |
| Aquasorb A500 | 34.0% | | | | 170.00 | |
| argan oil | 12.0% | | | | | 60.00 |
| | | | | | | |
| | | | | | | |
| | | | | | | |
| TOTAL | 100% | | 170.00 | 100.00 | 170.00 | 60.00 |
| MIXING TIME (min) | 36 | | 3 | 3 | 30 | |

**Table 12 - Formulation J**

| RAW MATERIAL | TOTAL | | PHASE 1 | PHASE 2 | PHASE 3 | PHASE 4 |
|---|---|---|---|---|---|---|
| | | | | | | |
| Oppanol B12 | 0.0% | | 0 | | | |
| T2561 | 34.0% | | 170.00 | | | |
| T2560 | 20.0% | | | 100.00 | | |
| Aquasorb A500 | 34.0% | | | | 170.00 | |
| avocado oil | 12.0% | | | | | 60.00 |
| | | | | | | |
| | | | | | | |
| | | | | | | |
| TOTAL | 100% | | 170.00 | 100.00 | 170.00 | 60.00 |
| MIXING TIME (min) | 36 | | 3 | 3 | 30 | |

**Table 13 - Formulation K**

| RAW MATERIAL | TOTAL | | PHASE 1 | PHASE 2 | PHASE 3 | PHASE 4 |
|---|---|---|---|---|---|---|
| | | | | | | |
| Oppanol B12 | 0.0% | | 0 | | | |
| T2561 | 34.0% | | 170.00 | | | |
| T2560 | 20.0% | | | 100.00 | | |
| Aquasorb A500 | 34.0% | | | | 170.00 | |
| jojoba oil | 12.0% | | | | | 60.00 |
| | | | | | | |
| | | | | | | |
| | | | | | | |
| TOTAL | 100% | | 170.00 | 100.00 | 170.00 | 60.00 |
| MIXING TIME (min) | 36 | | 3 | 3 | 30 | |

**Table 14 - Formulation L**

| RAW MATERIAL | TOTAL | | PHASE 1 | PHASE 2 | PHASE 3 | PHASE 4 |
|---|---|---|---|---|---|---|
| | | | | | | |
| Oppanol B12 | 0.0% | | 0 | | | |
| T2561 | 14.0% | | 70.00 | | | |
| T2560 | 40.0% | | | 200.00 | | |
| Aquasorb A500 | 34.0% | | | | 170.00 | |
| amandel oil | 12.0% | | | | | 60.00 |
| | | | | | | |
| | | | | | | |
| | | | | | | |
| TOTAL | 100% | | 70.00 | 200.00 | 170.00 | 60.00 |
| MIXING TIME (min) | 36 | | 3 | 3 | 30 | |

After mixing through the various phases, the resulting composition was pressed at 60°C to a layer thickness of 0.5 mm.

A series of measurements were taken of samples formed from the compositions. Specifically, the samples were subjected to 90° peel adhesion tests, reverse tack tests, and static absorption tests. Each of these tests are as follows.

### 90° Peel Adhesion of Hydrocolloid Adhesives on Stainless Steel

Peel adhesion on stainless steel (SS) is the average force to remove a hydrocolloid adhesive, laminated under specified conditions on a stainless steel panel, from the stainless steel panel at constant speed and at an angle of 90°.

### Procedure

Clean the stainless steel panel with solvent. Cut a hydrocolloid sample of 25.4 mm width and reinforce with reinforcing tape. Laminate a paper strip at one end of the hydrocolloid sample using an overlap of about 1 cm. Remove the liner from the hydrocolloid sample and laminate the sample on the stainless steel panel with a 450 gm. Roller at a speed of 150 cm/min. Allow the sample to dwell for 1 minute. Place the paper strip in an upper clamp of the test system and the stainless steel panel in the lower clamp, making sure that the angle between peel direction and stainless steel panel is 90°. Start the measurement using a crosshead speed of 300 mm/min. The angle must be kept at 90° until the measurement is completed. The 90° peel adhesion is the average force to remove the hydrocolloid strip from the stainless steel panel.

### Reverse Tack

Reverse tack of hydrocolloid adhesives is the maximum force necessary to remove a standard polyester strip brought into contact with the hydrocolloid without external force, from this hydrocolloid surface.

### Procedure

Prepare the test panel using double coated self adhesive tape. Laminate the hydrocolloid adhesive on the test panel. Place the test panel with the hydrocolloid in the lower clamp. Program the tensile tester. Place a polyester test strip of thickness 125 µm (5 mils) and dimensions (21 cm x 2.54 cm) in the upper clamp, making sure that the total length of polyester under the clamp (loop) is 15 cm. Remove the release liner from the hydrocolloid and start the measurement.

The reverse tack is the maximum force to remove the polyester strip from the hydrocolloid surface.

### Static Shear of Hydrocolloid Adhesives

Static shear is the time necessary to remove a hydrocolloid adhesive, laminated on a stainless steel panel under specified conditions, from the test panel under influence of a specified weight.

### Procedure

Condition the hydrocolloid samples at 23 ± 1° C and 50 ± 2% relative humidity for 24 hours. Clean the stainless steel shear panel with solvent. Cut a hydrocolloid strip of 25.4 mm width and 50 mm length. Reinforce the hydrocolloid strip with reinforcing tape. Laminate the hydrocolloid strip on the test panel using an overlap surface of 1 inch². Protect the free hydrocolloid with release liner. Put a weight of 500 g on the laminate for 1 hour. Reinforce the free hydrocolloid adhesive zone with reinforcing plastic and perforate. Place the test panel with hydrocolloid on the shear bar using a shear weight of 500 g. Re-zero the registration clock. Note the time on the clock when the measurement is completed.

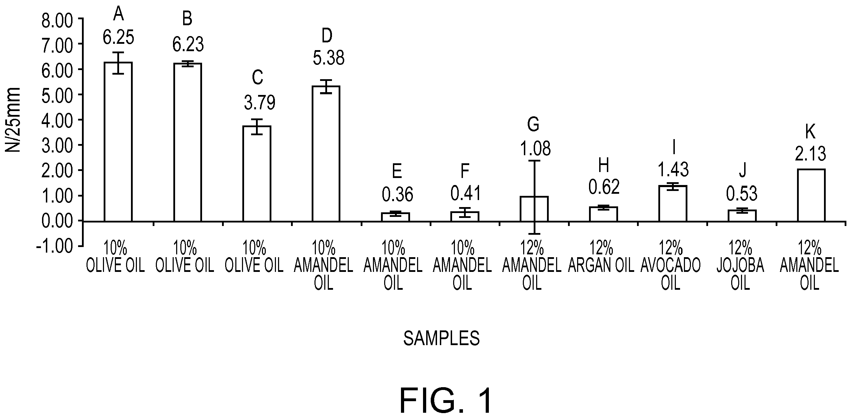

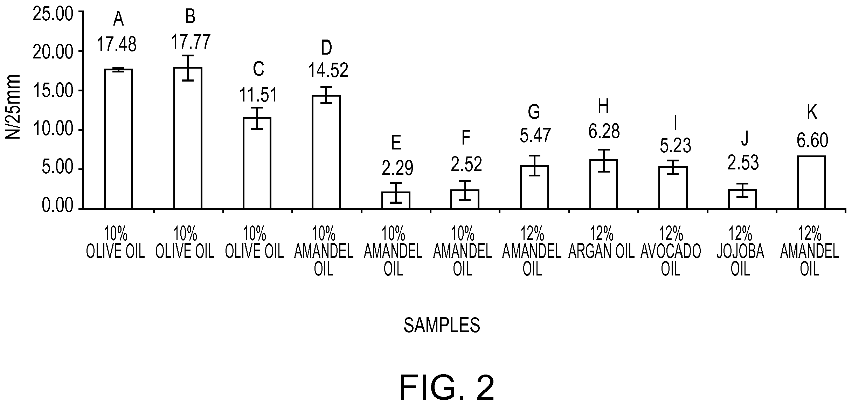

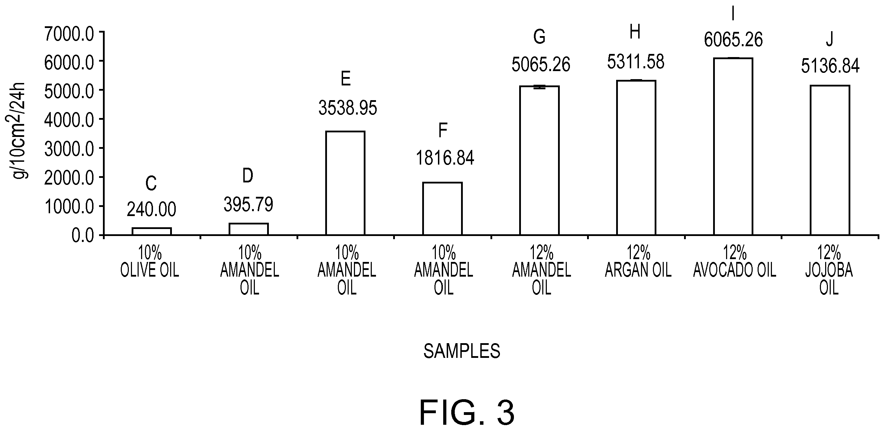

Table 15 lists a summary of adhesive performance. Figure 1 is a graph of 90° peel adhesion results of samples using the preferred embodiment compositions. Figure 2 is a graph of reverse tack results of samples using the preferred embodiment compositions. Figure 3 is a graph of static absorption of samples using the preferred embodiment composition.

**Table 15 - Summary of Adhesive Performance**

| Formulation Non-sterile Samples (laminated on PE) | 90° Peel Adhesion on Stainless Steel (N/25 mm) T04/142 | | | Reverse Tack (N/25 mm) T04/009 | | | Static Absorption (g/m²/7d) T06/022 | | |
|---|---|---|---|---|---|---|---|---|---|
| | results | average | st dev | results | average | st dev | results | average | st dev |
| | | | | | | | | | |
| A 10% olive oil | 5.84 | | | 17.78 | | | / | | |
| 17.5% Oppanol B12 | 6.66 | 6.25 | 0.41 | 17.43 | 17.48 | 0.28 | / | | |
| 37.5% T2560 35% A500 | 6.25 | | | 17.22 | | | / | | |
| | | | | | | | | | |
| C 10% olive oil | 6.13 | | | 18.92 | | | / | | |
| 60% T2560 | 6.25 | 6.23 | 0.10 | 15.95 | 17.77 | 1.59 | / | | |
| 30% A500 | 6.32 | | | 18.44 | | | / | | |
| | | | | | | | | | |
| D 10% olive oil | 3.55 | | | 12.72 | | | 240.00 | | |
| 30% T2561/30% T2560 | 3.70 | 3.79 | 0.30 | 10.23 | 11.51 | 1.25 | / | 240.00 | / |
| 30% A500 | 4.12 | | | 11.57 | | | / | | |
| | | | | | | | | | |
| E 10% amandel oil | 5.57 | | | 15.31 | | | 395.79 | | |
| 60% T2560 | 5.13 | 5.38 | 0.22 | 14.92 | 14.52 | 1.05 | / | 395.79 | / |
| 30%A500 | 5.43 | | | 13.33 | | | / | | |
| | | | | | | | | | |
| F 10% amandel oil | 0.40 | | | 2.56 | | | 3486.32 | | |
| 60% T2561 | 0.26 | 0.36 | 0.08 | 0.90 | 2.29 | 1.27 | 3591.58 | 3538.95 | 74.43 |
| 30% A500 | 0.41 | | | 3.40 | | | / | | |
| | | | | | | | | | |
| G 10% amandel oil | 0.52 | | | 2.06 | | | 1831.58 | | |
| 40% T2561/20% | | | | | | | | | |
| T2560 | 0.50 | 0.41 | 0.17 | 3.78 | 2.52 | 1.10 | 1802.11 | 1816.84 | 20.84 |
| 30% A500 | 0.21 | | | 1.73 | | | / | | |
| | | | | | | | | | |
| H 12% amandel oil | *3.79* | | | 5.14 | | | 5031.58 | | |
| 34% T2561/20% T2560 | *3.12* | 1.08 | 1.41 | 4.46 | 5.47 | 1.21 | 5098.95 | 5065.26 | 47.64 |
| 34% A500 | 1.08 | | | 6.82 | | | / | | |
| | | | | | | | | | |
| I 12% argan oil | 0.56 | | | 4.93 | | | 5309.47 | | |
| 34% T2561/20% T2560 | 0.59 | 0.62 | 0.07 | 7.70 | 6.28 | 1.39 | 5313.68 | 5311.58 | 2.98 |
| 34% A500 | 0.70 | | | 6.20 | | | / | | |
| | | | | | | | | | |
| J 12% avocado oil | 1.41 | | | 4.91 | | | 5974.74 | | |
| 34% T2561/20% T2560 | 1.56 | 1.43 | 0.13 | 4.60 | 5.23 | 0.83 | 6155.79 | 6065.26 | 128.02 |
| 34% A500 | 1.31 | | | 6.17 | | | / | | |
| | | | | | | | | | |
| K 12% jojoba oil | 0.60 | | | 1.98 | | | 4981.05 | | |
| 34% T2561/20% T2560 | 0.52 | 0.53 | 0.06 | 2.69 | 2.53 | 0.49 | 5292.63 | 5136.84 | 220.32 |
| 34% A500 | 0.48 | | | 2.93 | | | / | | |
| | | | | | | | | | |
| L 12% amandel oil | 2.19 | | | 7.50 | | | / | | |
| 14% T2561/40% T2560 | 2.17 | 2.13 | 0.08 | 5.50 | 6.60 | 1.01 | / | | |
| 34% A500 | 2.04 | | | 6.80 | | | / | | |

Formulation J with avocado oil exhibited the best properties, e.g., a combination of good adhesive performance and good static absorption. Generally, when utilizing T2560 and T2561 in the mixture and the ratio T2560/T2561 is 1:1 or higher, the formulation exhibits instability because the formulation leaches the hydrocolloid after water absorption.

Many other benefits will no doubt become apparent from future application and development of this technology.

It will be understood that any one or more feature or component of one embodiment described herein can be combined with one or more other features or components of another embodiment. Thus, the present subject matter includes any and all combinations of components or features of the embodiments described herein.

As described hereinabove, the present subject matter solves many problems associated with previous type compositions and practices. However, it will be appreciated that various changes in the details, materials and arrangements of components and operations, which have been herein described and illustrated in order to explain the nature of the subject matter, may be made by those skilled in the art without departing from the principle and scope of the subject matter, as expressed in the appended claims.

## Claims

1. A composition comprising, by weight:
(i) from 0.1 % to 20 % of at least one vegetable oil;
(ii) from 37.5 % to 60 % of hot melt adhesive(s) containing styrene-isoprene-styrene and styrene-isoprene block copolymers;
(iii) from 30 % to 35 % of one or more hydrocolloid(s); and
(iv) from 0 to 17.5 % of polyisobutene.

2. The composition of claim 1, wherein the hydrocolloid is sodium carboxymethyl cellulose.

3. A method of preparing a composition according to claim 1 including vegetable oil, the method comprising:
providing the components (i) to (iii) and optionally (iv) given in claim 1 in the amounts given in claim 1, and
blending component (i) with components (ii) and (iii) and optionally (iv) to thereby form the composition.

4. The method of claim 3, wherein the amount of component (i) is from 10 % to 20 % by weight of the total amount of the composition.

5. The method of claim 3 or 4, further comprising:
heating component (i) prior to or during blending.

6. The method of any one of claims 3 to 5, further comprising:
heating at least one of component (ii) and component (iii) prior to or during blending.

7. The method of any one of claims 3 to 6, wherein blending is performed incrementally.

8. An article adapted for placement on a user's skin, the article defining a skin-contacting region, wherein a composition of claim 1 or 2 is disposed on at least a portion of the skin-contacting region.

9. The article of claim 8, wherein the article is selected from the group consisting of consumer patches, consumer wound care products, dressings, bandages, and cosmetic patches.

## Patentansprüche

1. Zusammensetzung, umfassend, bezogen auf das Gewicht:
(i) von 0,1% bis 20% von mindestens einem pflanzlichen Öl;
(ii) von 37,5% bis 60% eines Heißschmelzklebers bzw. von Heißschmelzkleber, enthaltend Styrol-Isopren-Stryol und Styrol-Isopren-Blockcopolymere,
(iii) von 30% bis 35% von einem oder mehreren Hydrokolloiden, und
(iv) von 0 bis 17,5% Polyisobuten.

2. Zusammensetzung gemäß Anspruch 1, wobei das Hydrokolloid Natriumcarboxymethylcellulose ist.

3. Verfahren zur Herstellung einer Zusammensetzung gemäß Anspruch 1, welche pflanzliches Öl einschließt, wobei das Verfahren umfaßt:
das Bereitstellen der Komponenten (i) bis (iii) und gegebenenfalls (iv), wie in Anspruch 1 angegeben, in den Mengen, wie in Anspruch 1 angegeben, und
das Mischen der Komponente (i) mit Komponenten (ii) und (iii) und gegebenenfalls (iv), um dadurch die Zusammensetzung zu bilden.

4. Verfahren gemäß Anspruch 3, wobei die Menge der Komponente (i) von 10 Gew.-% bis 20 Gew.-%, bezogen auf die Gesamtmenge der Zusammensetzung, beträgt.

5. Verfahren gemäß Anspruch 3 oder 4, weiter umfassend:
das Erwärmen der Komponente (i) vor dem oder während des Mischens.

6. Verfahren gemäß einem der Ansprüche 3 bis 5, weiter umfassend:
das Erwärmen von mindestens einer der Komponente (ii) und Komponente (iii) vor dem oder während des Mischens.

7. Verfahren gemäß einem der Ansprüche 3 bis 6, wobei das Mischen taktweise durchgeführt wird.

8. Gegenstand, eingerichtet zur Anordnung auf der Haut eines Anwenders, wobei der Gegenstand einen Haut-kontaktierenden Bereich definiert, wobei eine Zusammensetzung gemäß Anspruch 1 oder 2 auf mindestens einem Abschnitt des Haut-kontaktierenden Bereichs angeordnet ist.

9. Gegenstand gemäß Anspruch 8, wobei der Gegenstand aus der Gruppe, bestehend aus Verbraucherpflaster, Verbraucherwundheilprodukten, Verbandsstoffen, Bandagen und Kosmetikpflastern, ausgewählt ist.

## Revendications

1. Une composition comprenant, en poids:
(i) de 0,1% à 20% d'au moins une huile végétale;
(ii) de 37,5% à 60% d'un ou de plusieurs adhésifs thermofusibles contenant des copolymères à blocs styrène-isoprène-styrène et styrène-isoprène;
(iii) de 30% à 35% d'un ou de plusieurs hydrocolloïdes; et
(iv) de 0 à 17,5% de polyisobutène.

2. La composition selon la revendication 1, dans laquelle l'hydrocolloïde est de la carboxyméthyl cellulose de sodium.

3. Un procédé de préparation d'une composition selon la revendication 1 comprenant de l'huile végétale, le procédé comprenant:
fournir les composants (i) à (iii) et optionnellement (iv) donnés à la revendication 1 dans les quantités indiquées à la revendication 1, et
mélanger le composant (i) avec les composants (ii) et (iii) et optionnellement (iv) pour ainsi former la composition.

4. Le procédé selon la revendication 3, dans lequel la quantité du composant (i) est de 10% à 20% en poids de la quantité totale de la composition.

5. Le procédé selon la revendication 3 ou 4, comprenant en outre:
le chauffage du composant (i) avant ou pendant le mélange.

6. Le procédé selon l'une quelconque des revendications 3 à 5, comprenant en outre:
le chauffage d'au moins un du composant (ii) et du composant (iii) avant ou pendant le mélange.

7. Le procédé selon l'une quelconque des revendications 3 à 6, dans lequel le mélange se fait par incréments.

8. Un article destiné à être placé sur la peau d'un utilisateur, l'article définissant une zone de contact avec la peau, dans lequel une composition selon la revendication 1 ou 2 est déposée sur au moins une partie de la zone de contact avec la peau.

9. L'article selon la revendication 8, dans lequel l'article est sélectionné parmi le groupe consistant en des patchs, produits de soin des blessures, pansements, bandages et patchs cosmétiques.
